# EUROPÄISCHE PATENTANMELDUNG

(11) **EP 1 848 185 A1**
(43) Veröffentlichungstag der Anmeldung: **24.10.2007**
(21) Anmeldenummer: 07103464.9
(22) Anmeldetag: 05.03.2007
(51) Int. Cl.: H04M 1/725, G08B 25/01

(54) **Telefonkombination aus Mobiltelefon und Zusatzgerät**

(30) Priorität: 18.04.2006 DE 202006006136 U
(71) Anmelder: LIFTAcom Telefondienste-GmbH & Co. KG, 50858 Köln (DE)
(72) Erfinder: Mayer, Günther, 27432, Iselersheim (DE)
(74) Vertreter: Selting, Günther

(57) **Zusammenfassung**

Ein Mobiltelefon, das mit einem Spracherkennungsteil (13) und einem Signalempfangsteil (14) ausgestattet ist, wird in Verbindung mit einem am Körper des Benutzers zu tragenden Zusatzgerät (15) verwendet. Wenn ein Bedienungselement (16) des Zusatzgerätes betätigt wird, wird am Mobiltelefon (10) die Spracherkennung aktiviert. Der Benutzer kann dann durch Sprechen des Namens der gewünschten Zielperson einen Wählvorgang auslösen. Der Vorteil liegt darin, dass das komplizierte Tastenfeld (11) des Mobiltelefons nicht benutzt werden muss. Vielmehr genügt ein einfacher Knopfdruck am Zusatzgerät, den Wahlvorgang vorzubereiten, bzw. auszuführen.

## Beschreibung

Die Erfindung betrifft eine Telefonkombination aus einem Mobiltelefon mit Spracherkennungsteil und einem Signalempfangsteil, und einem Zusatzgerät.

Üblich sind Mobilfunktelefone, die über eine Bluetooth-Überfiragungsstrecke von einem Zusatzgerät Signale empfangen können. Ferner haben moderne Mobilfunktelefone einen Spracherkennungsteil, der vom Benutzer gesprochene Namen erkennt und automatisch die betreffende Telefonnummer wählt, wenn der erkannte Name im Telefonverzeichnis gespeichert ist.

Ältere oder sehbehinderte Menschen sind oft nicht imstande, ein Mobilfunktelefon zu bedienen, weil dort zu viele Knöpfe und Funktionen vorhanden sind. Insbesondere in Notsituationen oder in einem Zustand der Erregung sind viele Menschen nicht imstande, eine Telefonverbindung durch Wählen herzustellen oder auch nur das Telefon in einen Zustand zu versetzen, in dem ein gesprochener Name zur Einleitung eines Wählvorganges führt. Andererseits brauchen ältere und behinderte Menschen die Möglichkeit, auch in Notsituationen bestimmte Personen oder eine Notrufzentrale anzurufen.

Der Erfindung liegt die Aufgabe zugrunde, eine Telefonkombination aus einem Mobiltelefon und einem Zusatzgerät zu schaffen, die mit einfachen Mitteln bedienbar ist, um selbst in Ausnahmesituationen ausgewählte Personen oder Institutionen zu benachrichtigen.

Die erfindungsgemäße Telefonkombination ist durch den Anspruch 1 definiert. Sie weist ein Mobiltelefon mit Spracherkennungsteil und Signalempfangsteil auf, und ein Zusatzgerät mit mindestens einem Bedienungselement und einem mit dem Signalempfangsteil des Mobiltelefons korrespondierenden Signalsendeteil, wobei eine Betätigung des Bedienungselements die Aktivierung des Spracherkennungsteils hervorruft.

Die Erfindung bietet den Vorteil, dass der Benutzer lediglich ein einfaches Bedienungselement des Zusatzgerätes betätigen muss, um das Mobiltelefon über dessen Spracherkennungsteil in einen Wählzustand zu versetzen. Der Benutzer braucht also beispielsweise nur einen einzigen Knopf am Zusatzgerät zu drücken und kann anschließend die Zieleingabe seines Anrufs durch Sprechen des Namens tätigen. Ein solcher Vorgang ist selbst in Zuständen besonderer Erregung oder Schockzuständen möglich, wenn ein komplexer Bedienungsvorgang am Mobiltelefon für die betreffende Person durchführbar wäre. Das Zusatzgerät kann ein Teil sein, das problemlos am Körper mitgeführt werden kann, beispielsweise an einem um den Hals zu tragenden Band oder einem Armband. Das Zusatzgerät weist vorzugsweise nur ein einziges Bedienungselement in Form einer Taste auf, so dass der Benutzer nicht eines von mehreren Bedienungselementen auswählen muss. Das Bedienungselement kann allerdings unterschiedliche Funktionen ausüben, beispielsweise durch einmaliges oder mehrmaliges Drücken oder auch durch längeres Drücken.

Gemäß einer bevorzugten Weiterbildung der Erfindung ist vorgesehen, dass eine Betätigung eines Bedienungselements des Zusatzgerätes das Wählen einer vorbestimmten Telefonnummer durch das Mobiltelefon veranlasst. In diesem Fall kann außer dem ersten Bedienungselement, das die Aktivierung des Spracherkennungsteils hervorruft, ein weiteres Bedienungselement für das Wählen einer vorbestimmten Telefonnummer vorhanden sein. Es besteht aber auch die Möglichkeit, beide Funktionen durch unterschiedliche Betätigungen eines einzigen Bedienungselementes wahrzunehmen. Der Benutzer hat die Möglichkeit, an dem Zusatzgerät durch einfache Betätigung des Bedienungselements eine Person seines Vertrauens anzurufen. Dies ist in Notfällen, beispielsweise bei einem Sturz, wichtig.

Ferner kann eine Betätigung des Bedienungselements das Absetzen eines Notrufs und das Verbinden mit einer Notrufzentrale veranlassen. In diesem Fall wird automatisch eine Voice-ID-Kennung des Mobiltelefons zu der Notrufzentrale übertragen, so dass der anrufende Benutzer in der Notrufzentrale identifiziert werden kann, auch wenn er nicht imstande ist, eine Nachricht zu sprechen.

Eine Weiterbildung der Erfindung sieht vor, dass das Mobiltelefon die Übertragung einer die betreffende Funkzelle identifizierenden Information veranlasst. Das Mobilfunknetz ist bekanntlich in regionale Funkzellen aufgeteilt, was die Benutzer beim Telefonieren allerdings nicht bemerken. Um die Stelle, von der der Anruf kommt, in der Notrufzentrale wenigstens grob zu identifizieren, wird eine Information über die betreffende Funkzelle, in deren Bereich sich das Mobilfunktelefon befindet, mitgesendet. Auf diese Weise ist es im Notfall möglich, den Anrufer leichter aufzufinden.

Anstelle einer Angabe über die Funkzelle, kann das Mobiltelefon mit einem Positionserkennungsteil, beispielsweise einem GPS-System, ausgestattet sein und seine Position an einen anderen Telefonteilnehmer senden. Auf diese Weise ist eine sehr genaue Positionsbestimmung des Ortes, von dem der Anruf kam, möglich.

Die Anwendung der Erfindung ist nicht auf ältere oder behinderte Menschen beschränkt. Der wesentliche Vorteil ist eine vereinfachte Bedienung des Telefongeräts und eine problemlose Durchführung des Wahlvorganges, auch in Situationen, in denen der Benutzer abgelenkt ist. So kann beispielsweise eine Servicezentrale angerufen werden, die für den Benutzer verschiedene Dienstleistungen ausführt.

Das Zusatzgerät führt eine drahtlose unidirektionale Signalübertragung zu dem Mobiltelefon durch. Diese Datenübertragung kann beispielsweise nach dem Bluetooth-Standard erfolgen oder auch durch andere Übertragungstechniken, z.B. durch Infrarot-Signale. Die maximale Entfernung für eine funktionsfähige Verbindung ist z. B. auf 10 Meter begrenzt.

Weiterhin besteht die Möglichkeit, die Telefonkombination mit bestimmten medizinischen Geräten zu kombinieren, um den Notfall-Service für gefährdete Personen direkt zugänglich zu machen. So kann beispielsweise ein Defibrillator mit dem Zusatzgerät ausgestattet sein. Wird er in Funktion gesetzt, wird das Freisprechmodul aktiviert und fragt den Benutzer sprachgesteuert, ob er Hilfe benötigt. Bejaht er dies, wird er unmittelbar mit dem Notfall-Center verbunden.

Unter einem Mobiltelefon wird sowohl ein Mobilfunktelefon verstanden als auch ein mobiles Telefonteil einer festen Installation. Die Mobilität ist Voraussetzung dafür, dass sich das Mobiltelefon stets in der Nähe des Benutzers befindet, so dass es mit dem Spracherkennungsteil auf die Sprache reagieren kann.

Im Folgenden wird unter Bezugnahme auf die Zeichnungen ein Ausführungsbeispiel der Erfindung näher erläutert.

Es zeigen:
- Figur 1: eine schematische Darstellung eines Mobiltelefons mit dem Zusatzgerät,
- Figur 2: eine Seitenansicht einer Ausführungsform des Zusatzgerätes und
- Figur 3: eine Draufsicht auf Figur 2 aus Richtung des Pfeils III.

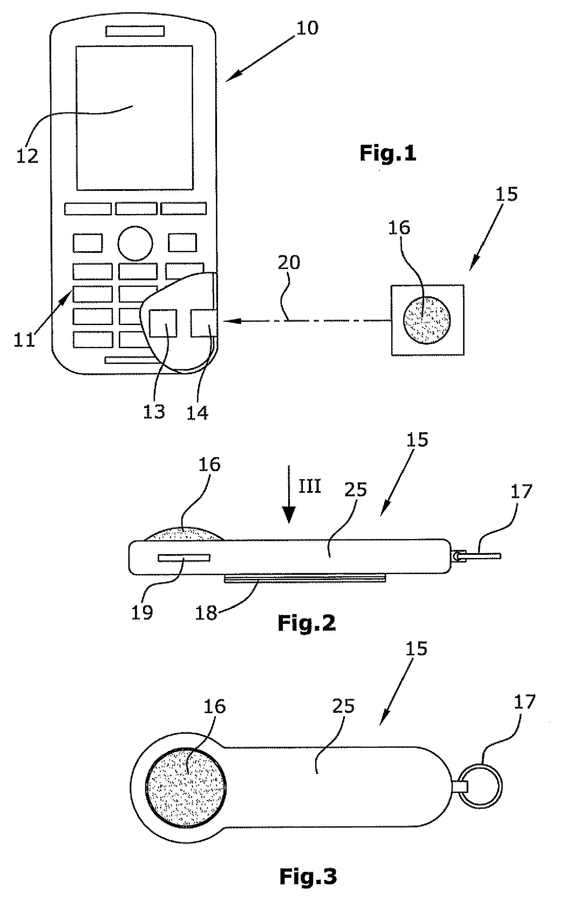

In Figur 1 ist ein übliches Mobiltelefon 10 dargestellt, das neben einem Tastenfeld 11 ein Display 12 aufweist. Das Mobiltelefon 10 enthält ferner einen Spracherkennungsteil 13, der imstande ist, ein gesprochenes Wort, z.B. einen Namen, zu erkennen und mit einem elektronischen Adressbuch zu kommunizieren, das diesen Namen einer Telefonnummer zuordnet. Selbstverständlich ist der Spracherkennungsteil 13 mit einem im Mobiltelefon vorgesehenen Mikrofon verbunden.

Das Mobiltelefon 10 enthält außerdem einen Signalempfangsteil 14, der mit einem externen Zusatzgerät 15 über eine drahtlose Übertragungsstrecke 20 kommuniziert, z.B. in Bluetooth-Technik. Das Zusatzgerät 15 enthält einen Signalsendeteil, dessen Signale von dem Signalempfangsteil 14 empfangen werden.

Der Signalsendeteil des Zusatzgeräts 15 wird durch ein Bedienungselement 16 in Funktion gesetzt. Das Bedienungselement 16 ist hier ein Knopf, der vom Benutzer gedrückt werden kann, um ein Signal an das Mobiltelefon 10 zu übertragen.

Eine Ausführungsform des Zusatzgerätes 15 ist in den Figuren 2 und 3 dargestellt. Das Zusatzgerät weist ein Gehäuse 25 auf, das nach Art eines Schlüssels länglich gestaltet ist. An dem einen Ende des Gehäuses 25 befindet sich ein Aufhänger 17 und am gegenüberliegenden Ende das Bedienungselement 16 in Form des Druckknopfes. Das Aufhängeelement 17 ist eine Öse, durch die ein Band gefädelt werden kann, das der Benutzer um den Hals trägt. Auf diese Weise ist ein ständiges Mitführen des Zusatzgerätes 15 möglich.

An der Unterseite des Gehäuses 25 befindet sich eine Führungsschiene 18, die in eine entsprechende Führung eines nicht dargestellten Armbandes eingesetzt werden kann. Auf diese Weise kann der Benutzer das Zusatzgerät wie eine Uhr am Handgelenk tragen.

Ferner ist eine Tastensperre 19 am Gehäuse 25 vorgesehen, mit der die Taste 16 gesperrt werden kann. Selbstverständlich enthält das Gehäuse 25 eine Batterie und den hier nicht dargestellten Signalsendeteil.

Außer dem Bedienungselement 16 enthält das Zusatzgerät 15 keine weiteren Bedienungselemente. Das Bedienungselement 16 bildet einen Multifunktionsschalter. Wird dieser beispielsweise zweimal kurz gedrückt, wird die Sprachwahlfunktion des Mobiltelefons 10 aktiviert, wodurch die Verbindung zum gewünschten Teilnehmer hergestellt wird.

Wird das Bedienungselement einmal kurz gedrückt, wird automatisch eine vorgegebene Telefonnummer gewählt, beispielsweise diejenige eines Verwandten oder einer Servicezentrale. Der Benutzer wird unmittelbar mit der gewählten Telefonnummer verbunden. Anhand einer mitübertragenen Voice-ID-Kennung des Benutzers ermittelt die Servicezentrale aus einer Benutzerdatenbank den vom jeweiligen Kunden gewählten Tarif und leitet das Gespräch an die entsprechende Gegenstelle weiter. Hier kann der Kunde eine numerische Sprachwahl tätigen. Er kann auch direkt seine Adressbucheinträge anwählen.

Drückt der Benutzer das Bedienungselement 16 einmal lang, so wird Notfallalarm ausgelöst. Das Zusatzgerät sendet dem Mobiltelefon den Befehl zur Wahl einer vorgegebenen Telefonnummer. Zusätzlich werden die durch einen eingebauten GPS-Empfänger ermittelten Positionsdaten mitübertragen. Der Betreuer in der angewählten Notfallzentrale erhält sofort den Standort des Betroffenen sowie alle hinterlegten Daten, wie z.B. chronische Krankheitsbilder, Kontaktdaten des Hausarztes, bevorzugtes Krankenhaus, und andere Besonderheiten.

Die Erfindung ermöglicht es, mit Hilfe eines Mobiltelefons auch in Notsituationen die richtigen Maßnahmen zielgerecht einzuleiten.

## Patentansprüche

1. Telefonkombination aus einem Mobiltelefon (10) mit Spracherkennungsteil (13) und einem Signalempfangsteil (14), und einem am Körper zu tragenden Zusatzgerät (15), welches mindestens ein Bedienungselement (16) und einen mit dem Signalempfangsteil (14) korrespondierenden Signalsendeteil aufweist, wobei das Zusatzgerät (15) derart ausgebildet ist, dass eine Betätigung des Bedienungselements (16) die Aktivierung des Spracherkennungsteils (13) hervorruft.

2. Telefonkombination nach Anspruch 1, **dadurch gekennzeichnet, dass** das Zusatzgerät (15) derart ausgebildet ist, dass eine Betätigung eines Bedienungselements (16) des Zusatzgeräts (15) das Wählen einer vorbestimmten Telefonnummer durch das Mobiltelefon (10) veranlasst.

3. Telefonkombination nach Anspruch 1 oder 2, **dadurch gekennzeichnet, dass** das Zusatzgerät (15) derart ausgebildet ist, dass eine Betätigung des Bedienungselements (16) das Absetzen eines Notrufs und das Verbinden mit einer Notrufzentrale veranlasst.

4. Telefonkombination nach einem der Ansprüche 1-3, **dadurch gekennzeichnet, dass** das Mobiltelefon (10) derart ausgebildet ist, dass es die Übertragung einer ID-Kennung zu der Notrufzentrale veranlasst.

5. Telefonkombination nach Anspruch 3, **dadurch gekennzeichnet, dass** das Mobiltelefon (10) derart ausgebildet ist, dass es die Übertragung einer die betreffende Funkzelle identifizierenden Information veranlasst.

6. Telefonkombination nach einem der Ansprüche 1-5, **dadurch gekennzeichnet, dass** das Zusatzgerät (15) einen einzigen Taster als Bedienungselement (16) aufweist.

7. Telefonkombination nach einem der Ansprüche 1-6, **dadurch gekennzeichnet, dass** das Zusatzgerät (15) eine Tastensperre (19) aufweist.

8. Telefonkombination nach einem der Ansprüche 1-7, **dadurch gekennzeichnet, dass** das Zusatzgerät (15) eine Befestigungsvorrichtung (17) für die lösbare Anbringung an einem Träger aufweist.

9. Telefonkombination nach einem der Ansprüche 1-8, **dadurch gekennzeichnet, dass** das Mobiltelefon (10) mit einem Positionserkennungsteil ausgestattet ist und imstande ist, seine Position an einen anderen Telefonteilnehmer zu senden.
